# EUROPÄISCHE PATENTSCHRIFT

(11) **EP 1 222 914 B1**
(45) Veröffentlichungstag und Bekanntmachung des Hinweises auf die Patenterteilung: **06.12.2006**
(21) Anmeldenummer: 02000447.9
(22) Anmeldetag: 08.01.2002
(51) Int. Cl.: A61K 8/34, A61K 8/37, A61K 8/44, A61K 8/49, A61Q 17/04

(54) **Wässrige kosmetische und dermatologische Zubereitungen mit einem Gehalt an wasserlöslichen UV-Filtersubstanzen**
Aqueous cosmetic and dermatological compositions comprising water soluble UV-filters
Compositions aqueuses cosmetiques ou dermatologiques comprenant des filtres UV hydrosolubles

(30) Priorität: 10.01.2001 DE 10100721
(43) Veröffentlichungstag der Anmeldung: 17.07.2002
(73) Patentinhaber: Beiersdorf Aktiengesellschaft, 20245 Hamburg (DE)
(72) Erfinder: Warnke, Katja, Dr., 20257 Hamburg (DE); Kruse, Uta, 20149 Hamburg (DE)

(56) Entgegenhaltungen:
- EP-A- 1 197 200
- WO-A-00/78277
- DE-A- 19 643 237
- DE-A- 19 846 772
- US-A- 5 961 960

## Beschreibung

Die vorliegende Erfindung betrifft wäßrige kosmetische und dermatologische Zubereitungen mit einem Gehalt an wasserlöslichen UV-Filtersubstanzengemäß Anspruch 1.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (*P. M. Elias, Structure and Function of the Stratum Comeum Permeability Barrier, Drug Dev. Res. **13**, 1988*, *97-105*) beschreibt die Hornschicht als Zwei-Komponenten-System, ähnlich einer Ziegelsteinmauer (Ziegelstein-Mörtel-Modell). In diesem Modell entsprechen die Homzellen (Komeozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die vorliegende Erfindung betrifft ferner kosmetische und dermatologische Lichtschutzformulierungen. Weiterhin betrifft die vorliegende Erfindung Zubereitungen zur kosmetischen und dermatologischen Behandlung oder Prophylaxe erythematöser, entzündlicher, allergischer oder autoimmunreaktiver Erscheinungen, insbesondere Dermatosen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasem zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können den kosmetischen bzw. dermatologischen Formulierungen zusätzlich Antioxidantien und/oder Radikalfänger zugesetzt werden. So ist beispielsweise vorgeschlagen worden, Vitamin E, eine Substanz mit bekannter antioxidativer Wirkung in Lichtschutzformulierungen einzusetzen, allerdings bleibt hier die erzielte Wirkung weit hinter der erhofften zurück.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Je nachdem, in welchem Bereich des UV-Lichtes absorbiert wird, unterscheidet man UV-B-Filter, UV-A-Filter und Breitbandfilter (welche über den gesamten Bereich des UV-A und UV-B eine Filterwirkung zeigen). Durch entsprechende Auswahl des UV-Filters und seiner Konzentration im Sonnenschutzmittel hat man die Möglichkeit, den Grad der Abschirmung des UV-Lichtes zu beeinflussen. Für die Dosierung der Substanzen in den fertigen Formulierungen können die Extinktionswerte allerdings allenfalls eine Orientierungshilfe bieten, denn durch Wechselwirkungen mit Inhaltsstoffen der Formulierung oder der Haut selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die meisten Sonnenschutzmittel werden in Wassernähe oder bei sportlicher Betätigung (Schwitzen) angewendet, weshalb der Wasserfestigkeit solcher Formulierungen besondere Bedeutung beizumessen ist. Ein wasserfestes Sonnenschutzmittel schützt den Anwender nicht nur nach dem Baden, sondern bewahrt ihn auch während des Badens vor einem Sonnenbrand.

Um hohe Lichtschutzfaktoren bei gleichzeitiger sehr guter Wasserfestigkeit zu erreichen, sind W/O-Formulierungen von Vorteil. Allerdings weisen W/O-Emulsionen des Standes der Technik häufig unbefriedigende kosmetische Eigenschaften auf. Bei der Anwendung können entsprechende Zubereitungen auf der Haut einen fettigen, glänzenden und zum Teil klebrigen Eindruck hinterlassen und sich - insbesondere auf behaarter Haut - schwierig verteilen lassen. Im Einzelfall können daher derartige Zubereitungen sogar nicht vermarktungsfähig sein, da sie vom Verbraucher nicht akzeptiert bzw. negativ beurteilt werden.

Eine Aufgabe der vorliegenden Erfindung war daher, den Nachteilen des Standes der Technik Abhilfe zu schaffen

Wäßrige und meist auch alkoholhaltige Lösungen stellen die Basis für sogenannte Tonics dar. Solche sind Gesichtswässer, die meist zum Abschluß der Gesichtspflege aufgelegt werden, um dem Gesicht ein Frischegefühl und ein erfrischtes Aussehen zu verleihen. Neben Parfum und anderen Zusätzen enthalten Tonics in der Regel Feuchthaltemittel wie beispielsweise Glycerin.

Üblicherweise liegt der Alkoholgehalt von Gesichtswässem im Bereich von 10 - 20 Gew.-%. Ein höherer Gehalt an Ethanol wäre bisweilen wünschenswert, um bestimmte Rezepturbestandteile, beispielsweise Parfumbestandteile, besser lösen zu können, andererseits führt erhöhter Ethanolgehalt nicht selten zu Hautreizung zumindest bei empfindlicher Haut.

Wäßrige Tönungslösungen bieten entgegen herkömmlichen Produkte zum Grundieren/Tönen des Gesichts, Hals oder Dekolleté wie Make-up, Foundations oder getönte Tagescreme den entscheidenden Vorteil, daß sie zu einer natürlich wirkenden, aber nicht abdeckenden Hauttönung führen (auch Rouge), ohne nach dem Einziehen auf Kleider abzufärben oder nachzufetten.

Bisherige Tönungslösungen bieten aber keine zusätzlichen Eigenschaften wie UV-Schutz oder besondere Pflegeeigenschaften. Zusätzliche Inhaltsstoffe führen leicht zu einem Eintrüben oder Verfärben der klaren Lösung, bzw. sind in wässriger Lösung nicht stabil formulierbar.

Diesen Übelständen galt es Abhilfe zu schaffen.

Erstaunlicherweise werden diese Aufgaben gelöst durch wäßrige kosmetische Tönungslösungen oder Gesichtswasser enthaltend
(a) 0,05 - 15 Gew.-% 2-Phenylbenzimidazol-5-sulfonsäure bzw. eines oder mehrerer ihrer Salze und/oder 0,05 - 15 Gew.-% Phenylen-1,4-bis-(2-benzimidazyl)-3,3'-5,5' tetrasulfonsäure bzw. eines oder mehrerer ihrer Salze,
(b) eine oder mehrere Substanzen, gewählt aus der Gruppe, gewählt aus der Gruppe der wasserlöslichen Konservierungsmittel, insbesondere 3-lodo-2-propinyl-butylcarbamat, 4-Hydroxybenzoesäureester, Phenoxyethanol,
(c) 50 - 98,5 Gew.-% Wasser,
(d) 0 - 30 Gew.-% Ethanol
(e) 0,05 - 3,0 Gew.-% an einem oder mehreren wasserlöslichen Farbstoffen,
(f) gegebenenfalls weitere kosmetische Hilfs- und/oder Zusatzstoffe,
(g) wobei die Zubereitung in einem pH-Bereich von 4-8 vorliegt.

Erstaunlicherweise bilden die erfindungsgemäßen Zubereitungen eine stabile wäßrige Darreichungssform mit hohem Lichtschutzfaktor. Gleichzeitig werden die hautbefeuchtende und -pflegende Wirkung verbessert, ohne daß es zu einer Eintrübung des Produktes durch die Inhaltsstoffe kommt.

Die 2-Phenylbenzimidazol-5-sulfonsäure ist gekennzeichnet durch folgende Struktur:

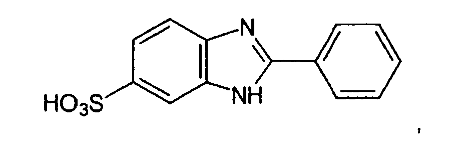

Die Phenylen-1,4-bis-(2-benzimidazyl)-3,3'-5,5'-tetrasulfonsäure ist durch folgende Struktur gekennzeichnet::

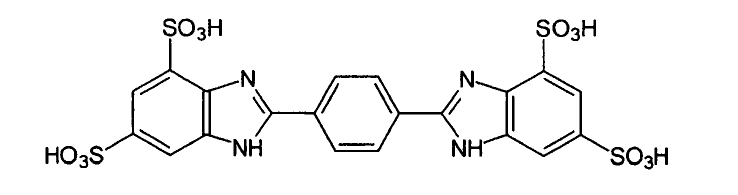

Beide Substanzen sind bekannte Lichtschutzfiltersubstanzen, wobei die 2-Phenylbenzimidazol-5-sulfonsäure überwiegend im UV-B-Bereich, die Phenylen-1,4-bis-(2-benzimidazyl)-3,3'-5,5'-tetrasulfonsäure überwiegend im UV-A-Bereich absorbiert.

Bevorzugt enthalten Zubereitungen gemäß der Erfindung 0,5 - 2,0 Gew.-% 2-Phenylbenzimidazol-5-sulfonsäure bzw. eines oder mehrerer ihrer Salze.

Ebenfalls bevorzugt enthalten Zubereitungen gemäß der Erfindung 0,5 - 1,0 Gew.-% Phenylen-1,4-bis-(2-benzimidazyl)-3,3'-5,5'-tetrasulfonsäure bzw. eines oder mehrerer ihrer Salze.

Es ist erfindungsgemäß von Vorteil, das Gewichtsverhältnis von 2-Phenylbenzimidazol-5-sulfonsäure zu Phenylen-1,4-bis-(2-benzimidazyl)-3,3'-5,5'-tetrasulfonsäure aus dem Bereich von 5 : 1 bis 1 : 5, bevorzugt aus dem Bereich von 3 : 1 bis 1 : 3, besonders bevorzugt ungefähr 2 : 1, zu wählen.

Phenoxyethanol ist vor allem in saurem und neutralem, aber auch im alkalischen Milieu wirksam und völlig ungiftig. Es gibt bereits in niedrigen Konzentrationen ausreichend Schutz. Aufgrund seines neutralen Geschmacks fand es schnell Eingang in die pharmazeutische und kosmetische Industrie. Seine Wirkung richtet sich allerdings hauptsächlich gegen gram-negative Bakterien.

Phenoxyethanol ist durch die chemische Struktur 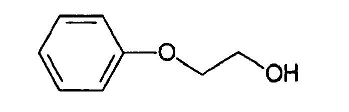 gekennzeichnet und stellt eine viskose Flüssigkeit von schwachem, leicht angenehmen Geruch und einem zusammenziehenden Geschmack dar. Phenoxyethanol wurde in der Natur nachgewiesen in tropischen Früchten, in Cichorium endivia sowie in grünem Tee (Camellia sinesis). Es hat einen milden, rosenähnlichen Duft und wird für Parfümkompositionen auch als Fixatur eingesetzt. Es ist mischbar mit Aceton, Ethylalkohol und Glycerin, löslich in Wasser und Fetten, z.B. Oliven- und Erdnußöl.

Erfindungsgemäß wird Phenoxyethanol in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,02 - 10,0 Gew.-%, besonders bevorzugt 0,02 - 5,0 Gew.-% an Phenoxyethanol, ganz besonders vorteilhaft 0,25 - 3,0 Gew.-%, bezogen auf das Gesamtgewicht der Zusammensetzung.

4-Hydroxybenzoesäureester (Paraben-Ester, PHB-Ester), werden in freier Form oder als Natrium-Salze zur Konservierung von Fisch- und Fleischerzeugnissen, Mayonnaisen, Salatsaucen, kosmetischen Präparaten, Arzneimitteln usw. verwendet.

Die als Konservierungsmittel wichtigsten 4-Hydroxybenzoesäureester sind 4-Hydroxybenzoesäuremethylester, 4-Hydroxybenzoesäureethylester, 4-Hydroxybenzoesäurepropylester, 4-Hydroxybenzoesäurebutylester. Der Methylester wurde auch als Sexuallockstoff läufiger Hündinnen identifiziert.

Die oft nur Methyl-, Ethyl-, Propyl-, Butyl- und Isobutylparaben genannten 4-Hydroxybenzoesäureester sind in Alkohol und Ether löslich, in Alkalilaugen lösen sie sich unter Salzbildung. Die Wirkung der Ester ist direkt proportional zur Kettenlänge des AlkylRestes, umgekehrt nimmt jedoch die Löslichkeit mit steigender Kettenlänge ab. Als nichtdissoziierende Verbindungen sind die Ester weitgehend pH-Wert-unabhängig und wirken in einem pH-Bereich von 3,0-8,0. Der antimikrobielle Wirkmechanismus beruht auf einer Schädigung der Mikrobenmembranen durch die Oberflächenaktivität der PHB-Ester sowie auf der Eiweiß-Denaturierung. Daneben treten Interaktionen mit Coenzymen auf. Die Wirkung richtet sich gegen Pilze, Hefen und Bakterien. Da PHB-Ester bereits ab einer Konzentration von ca. 0,08% in den Lebensmitteln zu geschmacklichen Beeinträchtigungen führen, ist die Verwendung in Lebensmitteln stark rückläufig.

Erfindungsgemäß bevorzugte 4-Hydroxybenzoesäureester sind 4-Hydroxybenzoesäuremethylester, 4-Hydroxybenzoesäureethylester, 4-Hydroxybenzoesäurepropylester, 4-Hydroxybenzoesäureisopropylester, 4-Hydroxybenzoesäurebutylester, 4-Hydroxybenzoesäureisobutylester.

Erfindungsgemäß werden der oder die 4-Hydroxybenzoesäureester in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,02 - 10,0 Gew.-%, besonders bevorzugt 0,02 - 5,0 Gew.-% an einem oder mehreren 4-Hydroxybenzoesäureestem, ganz besonders vorteilhaft 0,1 - 3,0 Gew.-%, jeweils bezogen auf das Gesamtgewicht der Zusammensetzung.

3-lodo-2-propinyl-butylcarbamat ist gekennzeichnet durch die chemische Strukturformel

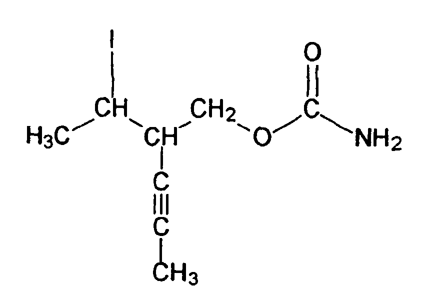

Erfindungsgemäß wird 3-lodo-2-propinyl-butylcarbamat in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,01 - 10,0 Gew.-%, besonders bevorzugt 0,01 - 5,0 Gew.-% an 3-lodo-2-propinyl-butylcarbamat, ganz besonders vorteilhaft 0,01 - 3,0 Gew.-%, bezogen auf das Gesamtgewicht der Zusammensetzung.

Ein weiteres vorteilhaftes wasserlösliches Konservierungsmittel im Sinne der vorliegenden Erfindung ist Hexamidineisethionat (4.4'-Diarnidino-α.ω-diphenoxyhexanisethionat).

Hexamidineisethionat ist gekennzeichnet durch die chemische Strukturformel

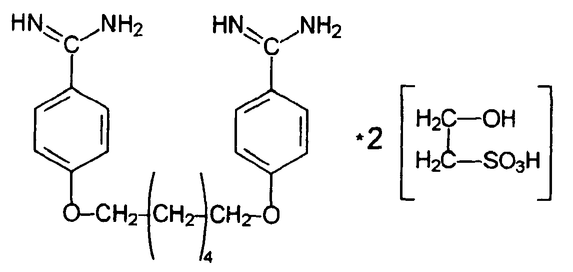

Erfindungsgemäß vorteilhaft wird Hexamidineisethionat in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,02 - 10,0 Gew.-%, besonders bevorzugt 0,02 - 5,0 Gew.-% an Phenoxyethanol, ganz besonders vorteilhaft 0,05 - 0,15 Gew.-%, bezogen auf das Gesamtgewicht der Zusammensetzung.

Ein weiteres vorteilhaftes wasserlösliches Konservierungsmittel im Sinne der vodiegenden Erfindung ist DMDM Hydantoin (1,3-Dimethylol-5,5-dimethylhydantoin).

DMDM Hydantoin ist gekennzeichnet durch die chemische Strukturformel

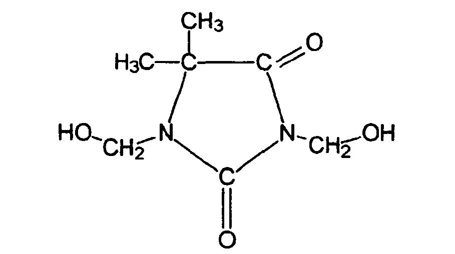

Erfindungsgemäß vorteilhaft wird DMDM Hydantoin in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,02 - 10,0 Gew.-%, besonders bevorzugt 0,02 - 5,0 Gew.-% an Phenoxyethanol, ganz besonders vorteilhaft 0,2 - 0,7 Gew.-%, bezogen auf das Gesamtgewicht der Zusammensetzung.

Ein weiteres vorteilhaftes wasserlösliches Konservierungsmittel im Sinne der vorliegenden Erfindung ist Diazolidinylharnstoff (N-[1,3-bis(hydroxymethyl)-2,5-dioxo-4-imidazolidinyl]-N.N'-bis(hydroxymethyl)harnstoff).

Diazolidinylharnstoff ist gekennzeichnet durch die chemische Strukturformel

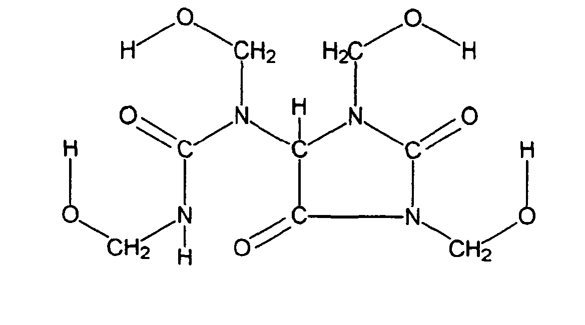

Erfindungsgemäß vorteilhaft wird Diazolidinyl Harnstoff in kosmetischen oder dermatologischen Zusammensetzungen eingesetzt, einem Gehalt 0,01 - 20,0 Gew.-%, bezogen auf das Gesamtgewicht der Zusammensetzung entsprechend. Vorteilhaft enthalten die Zusammensetzungen 0,02 - 10,0 Gew.-%, besonders bevorzugt 0,02 - 5,0 Gew.-% an Phenoxyethanol, ganz besonders vorteilhaft 0,1 - 0,3 Gew.-%, bezogen auf das Gesamtgewicht der Zusammensetzung.

Die erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol oder insbesondere Glycerin.

Insbesondere vorteilhaft enthalten Zubereitungen gemäß der vorliegenden Erfindung 0,5 - 25 Gew.-% Ethanol, bevorzugt 1 - 10 Gew.-% Ethanol, bezogen auf das Gesamtgewicht der Zubereitungen.

Insbesondere vorteilhaft enthalten Zubereitungen gemäß der vorliegenden Erfindung 0,5 - 25 Gew.-% Glycerin, bevorzugt 1 - 10 Gew.-% Glycerin, bezogen auf das Gesamtgewicht der Zubereitungen. Es kann erfindungsgemäß vorteilhaft sein, den erfindungsgemäßen Zubereitungen außer oder neben Glycerin noch ein oder mehrere Feuchthaltemittel zuzufügen.

Die kosmetischen Zubereitungen im Sinne der vorliegenden Erfindung können auch gewünschtenfalls Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder - distearat, enthalten, sowie insbesondere ein oder mehrere Verdickungsmittel, welches oder welche vorteilhaft gewählt werden können aus der Gruppe Siliciumdioxid, Polysaccharide bzw. deren Derivate, z.B. Hyaluronsäure, Xanthangummi, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Das Verdickungsmittel ist in dem Gel z.B. in einer Menge zwischen 0,1 und 30 Gew.-%, bevorzugt zwischen 0,5 und 15 Gew.-%, enthalten.

Die erfindungsgemäßen Zubereitungen können einen oder mehrere Farbstoffe und/oder Farbpigmente enthalten. Es ist erfindungsgemäß bevorzugt, daß solche Farbstoffe und/oder Farbpigmente wasserlöslich oder wasserdispergierbar sind. Es kann gegebenenfalls vorteilhaft sein, den erfindungsgemäßen Zubereitungen zusätzlich einen oder mehrere öllösliche Farbstoffe zuzufügen.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfonsäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1 '-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfonsäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxy-naphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 807 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,T-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Es kann femer günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Die erfindungsgemäßen Zubereitungen werden als Tönungsmittel für die Haut, insbesondere Gesichtshaut einzusetzen oder als Gesichtswasser zu verwenden.

Die nachfolgenden Beispiele sollen die vorliegende Erfindung verdeutlichen.

### Beispiel 1

| | Gew.-% |
|---|---|
| Butylenglycol | 10,00 |
| Propylenglycol, Maris limus, Ostrea | 2,00 |
| 2-Phenylbenzimidazol-5-sulfonsäure | 1,00 |
| Phenylen-1,4-bis-(2-benzimidazyl)-3,3'-5,5'-tetrasulfonsäure | 1,00 |
| PEG-40 Hydriertes Ricinusöl | 1,00 |
| C114720, CI19140, C128440 | 0,80 |
| Fucogel® 1000 | 0,50 |
| Phenoxyethanol | 0,40 |
| Methylparaben | 0,20 |
| 3-lodo-2-propinyl-butylcarbamat | 0,10 |
| Parfum | q.s. |
| Wasser | ad 100,00 |

### Beispiel 2

| | Gew.-% |
|---|---|
| Ethanol | 12,00 |
| Phenylen-1,4-bis-(2-benzimidazyl)-3,3'-5,5'-tetrasulfonsäure | 10,00 |
| 2-Phenylbenzimidazol-5-sulfonsäure | 8,00 |
| Glycerin | 5,00 |
| Galactoarabian | 1,00 |
| Cl 16255, Cl 15985, Cl 27755 | 0,20 |
| Phenoxyethanol | 0,20 |
| Methylparaben | 0,10 |
| 3-lodo-2-propinyl-butylcarbamat | 0,05 |
| Parfum | q.s. |
| Wasser | ad 100,00 |

### Beispiel 3

| | Gew.-% |
|---|---|
| Glycerin | 5,00 |
| PEG-150 | 2,50 |
| 2-Phenylbenzimidazol-5-sulfonsäure | 1,00 |
| Phenylen-1,4-bis-(2-benzimidazyl)-3,3'-5,5'-tetrasulfonsäure | 1,00 |
| Carbomer | 0,70 |
| CI14720, CI19140, CI28440 | 0,60 |
| Phenoxyethanol | 0,45 |
| Xylitol | 0,25 |
| Diazolidinylharnstoff | 0,25 |
| Natriumhyaluronat | 0,05 |
| Wasser | ad 100,00 |

### Beispiel 4

| | Gew.-% |
|---|---|
| Butylenglycol | 6,00 |
| Phenylen-1,4-bis-(2-benzimidazyl)-3,3'-5,5'-tetrasulfonsäure | 6,00 |
| 2-Phenylbenzimidazol-5-sulfonsäure | 4,00 |
| Hydroxyethylcellulose | 0,50 |
| Fucogel® 1000 | 0,30 |
| Cl 16255, Cl 15985, Cl 27755 | 0,30 |
| DMDM Hydantoin | 0,30 |
| Parfüm | q.s. |
| Wasser | ad 100,00 |

## Patentansprüche

1. Wäßrige kosmetische Tönungslösungen oder Gesichtswässer, enthaltend
(a) 0,05 - 15 Gew.-% 2-Phenylbenzimidazol-5-sulfonsäure bzw. eines oder mehrerer ihrer Salze und/oder 0,05 - 15 Gew.-% Phenylen-1,4-bis-(2-benzimidazyl)-3,3'-5,5'-tetrasulfonsäure bzw, eines oder mehrerer ihrer Salze,
(b) eine oder mehrere Substanzen, gewählt aus der Gruppe, gewählt aus der Gruppe der wasserlöslichen Konservierungsmittel, insbesondere 3-lodo-2-propinyl-butylcarbamat, 4-Hydroxybenzoesäureester, Phenoxyethanol,
(c) 50 - 98,5 Gew.-% Wasser,
(d) 0 - 30 Gew.-% Ethanol
(e) 0,05 - 3,0 Gew.-% an einem oder mehreren wasserlöslichen und/oder wasserdispergierbaren Farbstoffen,
(f) gegebenenfalls weitere kosmetische Hilfs- und/oder Zusatzstoffe,
(g) wobei die Zubereitung in einem pH-Bereich von 4-8 vorliegt.

2. Zubereitungen nach Anspruch 1, enthaltend 0,5 - 2,0 Gew.-% 2-Phenylbenzimidazol-5-sulfonsäure bzw. eines oder mehrerer ihrer Salze.

3. Zubereitungen nach Anspruch 1, enthaltend 0,5 - 1,0 Gew.-% Phenylen-1,4-bis-(2-benzimidazyl)-3,3'-5,5'-tetrasulfonsäure bzw. eines oder mehrerer ihrer Salze.

4. Zubereitungen nach Anspruch 1, **dadurch gekennzeichnet, daß** ein Gewichtsverhältnis von 2-Phenylbenzimidazol-5-sulfonsäure zu Phenylen-1,4-bis-(2-benzimidazyl)-3,3'-5,5'-tetrasulfonsäure aus dem Bereich von 5 : 1 bis 1 : 5, bevorzugt aus dem Bereich von 3 : 1 bis 1 : 3, besonders bevorzugt ungefähr 2 : 1, gewählt wird.

5. Zubereitungen nach Anspruch 1, **dadurch gekennzeichnet, daß** der oder die 4-Hydroxybenzoesäureester aus der Gruppe 4-Hydroxybenzoesäuremethylester, 4-Hydroxybenzoesäureethylester, 4-Hydroxybenzoesäurepropylester, 4-Hydroxybenzoesäureisopropylester, 4-Hydroxybenzoesäurebutylester, 4-Hydroxybenzoesäureisobutylester gewählt werden.

6. Zubereitungen nach Anspruch 1, **dadurch gekennzeichnet, daß** der oder die 4-Hydroxybenzoesäureester in Konzentrationen von 0,01 - 20,0 Gew.-%, bevorzugt 0,02 - 10,0 Gew.-%, besonders bevorzugt 0,02 - 5,0 Gew.-% an einem oder mehreren 4-Hydroxybenzoesäureestern, ganz besonders vorteilhaft 0,1 - 3,0 Gew.-%, vorliegen, jeweils bezogen auf das Gesamtgewicht der Zusammensetzung.

7. Zubereitungen nach Anspruch 1, **dadurch gekennzeichnet, daß** Phenoxyethanol in Konzentrationen von 0,01 - 20,0 Gew.-%, bevorzugt 0,02 - 10,0 Gew.-%, besonders bevorzugt 0,02 - 5,0 Gew.-% an Phenoxyethanol ganz besonders vorteilhaft 0,25 - 3,0 Gew.-%, vorliegt, jeweils bezogen auf das Gesamtgewicht der Zusammensetzung.

8. Zubereitungen nach Anspruch 1, **dadurch gekennzeichnet, daß** 3-lodo-2-propinyl-butylcarbamat in Konzentrationen von 0,01 - 20,0 Gew.-%, bevorzugt 0,02 - 10,0 Gew.-%, besonders bevorzugt 0,02 - 5,0 Gew.-% an 3-lodo-2-propinyl-butylcarbamat ganz besonders vorteilhaft 0,01 - 3,0 Gew.-%, vorliegt, jeweils bezogen auf das Gesamtgewicht der Zusammensetzung.

## Claims

1. Aqueous cosmetic toning solutions or face tonics, comprising
(a) 0.05-15% by weight of 2-phenylbenzimidazole-5-sulphonic acid or one or more of its salts and/or 0.05-15% by weight of phenylene-1,4-bis(2-benzimidazyl)-3,3'-5,5'-tetrasulphonic acid or one or more of its salts,
(b) one or more substances chosen from the group of water-soluble preservatives, in particular 3-iodo-2-propynyl butylcarbamate, 4-hydroxybenzoic ester, phenoxyethanol,
(c) 50-98.5% by weight of water,
(d) 0-30% by weight of ethanol
(e) 0.05-3.0% by weight of one or more water-soluble and/or water-dispersible dyes,
(f) optionally further cosmetic auxiliaries and/or additives,
(g) where the preparation is in a pH range of 4-8.

2. Preparations according to Claim 1, comprising 0.5-2.0% by weight of 2-phenylbenzimidazole-5-sulphonic acid or one or more of its salts.

3. Preparations according to Claim 1, comprising 0.5-1.0% by weight of phenylene-1,4-bis(2-benzimidazyl)-3,3'-5,5'-tetrasulphonic acid or one or more of its salts.

4. Preparations according to Claim 1, **characterized in that** a weight ratio of 2-phenylbenzimidazole-5-sulphonic acid to phenylene-1,4-bis(2-benzimidazyl)-3,3'-5,5'-tetrasulphonic acid is chosen from the range from 5:1 to 1:5, preferably from the range from 3:1 to 1:3, particularly preferably approximately 2:1.

5. Preparations according to Claim 1, **characterized in that** the 4-hydroxybenzoic ester or esters are chosen from the group methyl 4-hydroxybenzoate, ethyl 4-hydroxybenzoate, propyl 4-hydroxybenzoate, isopropyl 4-hydroxybenzoate, butyl 4-hydroxybenzoate, isobutyl 4-hydroxybenzoate.

6. Preparations according to Claim 1, **characterized in that** the 4-hydroxybenzoic ester or esters are present in concentrations of 0.01-20.0% by weight, preferably 0.02-10.0% by weight, particularly preferably 0.02-5.0% by weight, of one or more 4-hydroxybenzoic esters, very particularly advantageously 0.1-3.0% by weight, in each case based on the total weight of the composition.

7. Preparations according to Claim 1, **characterized in that** phenoxyethanol is present in concentrations of 0.01-20.0% by weight, preferably 0.02-10.0% by weight, particularly preferably 0.02-5.0% by weight, of phenoxyethanol, very particularly advantageously 0.25-3.0% by weight, in each case based on the total weight of the composition.

8. Preparations according to Claim 1, **characterized in that** 3-iodo-2-propynyl butylcarbamate is present in concentrations of 0.01-20.0% by weight, preferably 0.02-10.0% by weight, particularly preferably 0.02-5.0% by weight, of 3-iodo-2-propynyl butylcarbamate, very particularly advantageously 0.01-3.0% by weight, in each case based on the total weight of the composition.

## Revendications

1. Solutions de nuançage ou lotions faciales cosmétiques aqueuses, contenant
(a) 0,05 - 15 % en poids d'acide 2-phénylbenzimidazole-5-sulfonique ou d'un ou plusieurs de ses sels et/ou 0,05 - 15 % en poids d'acide phénylène-1,4-bis(2-benzimidazyl)-3,3'-5,5'-tétrasulfonique ou d'un ou plusieurs de ses sels,
(b) une ou plusieurs substances choisies dans le groupe des conservateurs hydrosolubles, en particulier le carbamate de 3-iodo-2-propynylbutyle, les esters d'acide 4-hydroxybenzoïque, le phénoxyéthanol,
(c) 50 - 98,5 % en poids d'eau,
(d) 0 - 30 % en poids d'éthanol,
(e) 0,05 - 3,0 % en poids d'un ou plusieurs colorants hydrosolubles et/ou dispersables dans l'eau,
(f) éventuellement d'autres adjuvants et/ou additifs cosmétiques,
(g) la préparation se trouvant dans un intervalle de pH de 4 - 8.

2. Préparations selon la revendication 1, contenant 0,5 - 2,0 % en poids d'acide 2-phénylbenzimidazole-5-sulfonique ou d'un ou plusieurs de ses sels.

3. Préparations selon la revendication 1, contenant 0,5 - 1,0 % en poids d'acide phénylène-1,4-bis(2-benzimidazyl)-3,3'-5,5'-tétrasulfonique ou d'un ou plusieurs de ses sels.

4. Préparations selon la revendication 1, **caractérisées en ce qu'**on choisit un rapport pondéral de l'acide 2-phénylbenzimidazole-5-sulfonique à l'acide phénylène-1,4-bis(2-benzimidazyl)-3,3'-5,5'-tétrasulfonique dans la plage allant de 5:1 à 1:5, de préférence dans la plage allant de 3:1 à 1:3, de façon particulièrement préférée d'environ 2:1.

5. Préparations selon la revendication 1, **caractérisées en ce que** le ou les esters d'acide 4-hydroxybenzoïque sont choisis parmi le 4-hydroxybenzoate de méthyle, le 4-hydroxybenzoate d'éthyle, le 4-hydroxybenzoate de propyle, le 4-hydroxybenzoate d'isopropyle, le 4-hydroxybenzoate de butyle, le 4-hydroxybenzoate d'isobutyle.

6. Préparations selon la revendication 1, **caractérisées en ce que** le ou les esters d'acide 4-hydroxybenzoïque est(sont) présent(s) à des concentrations de 0,01 - 20,0 % en poids, de préférence de 0,02 - 10,0 % en poids, de façon particulièrement préférée de 0,02 - 5,0 % en poids d'un ou plusieurs esters d'acide 4-hydroxybenzoïque, de façon tout particulièrement avantageuse de 0,1 - 3,0 % en poids, chaque fois par rapport au poids total de la composition.

7. Préparations selon la revendication 1, **caractérisées en ce que** le phénoxyéthanol est présent à des concentrations de 0,01 - 20,0 % en poids, de préférence de 0,02 -10,0 % en poids, de façon particulièrement préférée de 0,02 - 5,0 % en poids de phénoxyéthanol, de façon tout particulièrement avantageuse de 0,25 - 3,0 % en poids, chaque fois par rapport au poids total de la composition.

8. Préparations selon la revendication 1, **caractérisées en ce que** le carbamate de 3-iodo-2-propynyl-butyle est présent à des concentrations de 0,01 - 20,0 % en poids, de préférence de 0,02 - 10,0 % en poids, de façon particulièrement préférée de 0,02 - 5,0 % en poids de carbamate de 3-iodo-2-propynyl-butyle, de façon tout particulièrement avantageuse de 0,01 - 3,0 % en poids, chaque fois par rapport au poids total de la composition.
